# EUROPEAN PATENT APPLICATION

(11) **EP 1 298 141 A1**
(43) Date of publication of application: **02.04.2003**
(21) Application number: 01123096.8
(22) Date of filing: 27.09.2001
(51) Int. Cl.: C07K 14/54, A61K 38/20

(54) **Interleukin-4 (IL-4) promoter sequences specifically interacting with IRF-1 and IRF-2**

(71) Applicant: Deutsches Krebsforschungszentrum Stiftung des öffentlichen Rechts, 69120 Heidelberg (DE)
(72) Inventor: The designation of the inventor has not yet been filed
(74) Representative: Schüssler, Andrea, Dr.

(57) **Abstract**

Described are nucleic acid molecules originally located within the IL-4 promoter which specifically interact with IRF-1 and/or IRF-2 and pharmaceutical compositions containing said nucleic acid molecules.

SEQUENCE LISTING

<110> Deutsches Krebsforschungszentrum

<120> IL-4 promoter sequences specifically interacting with
      IRF-1 and IRF-2

<130> K3012

<140> EP 01 123 096.8
<141> 2001-09-27

<160> 12

<170> PatentIn Ver. 2.1

<210> 1
<211> 20
<212> DNA
<213> Artificial Sequence

<220>
<223> Description of Artificial Sequence:
      nucleic acid sequence interacting with IRF1 and/or IRF2

<400> 1
tggccccaag tgactgacaa                                              20


<210> 2
<211> 36
<212> DNA
<213> Artificial Sequence

<220>
<223> Description of Artificial Sequence:
      nucleic acid sequence interacting with IRF1 and/or IRF2

<400> 2
ctatgcaaag caaaaagcca gcagcagccc caagct                            36


<210> 3
<211> 20
<212> DNA
<213> Artificial Sequence

<220>
<223> Description of Artificial Sequence:
      Oligonucleotide

<400> 3
tggccccaag tgactgacaa                                              20


<210> 4
<211> 20
<212> DNA
<213> Artificial Sequence

<220>
<223> Description of Artificial Sequence:
      Oligonucleotide

<400> 4

aaaggtttca ttttcctatt 20

<210> 5
<211> 36
<212> DNA
<213> Artificial Sequence

<220>
<223> Description of Artificial Sequence:
      Oligonucleotide

<400> 5
ctatgcaaag caaaaagcca gcagcagccc caagct 36

<210> 6
<211> 18
<212> DNA
<213> Artificial Sequence

<220>
<223> Description of Artificial Sequence:
      Oligonucleotide

<400> 6
tcactttcac tttcactt 18

<210> 7
<211> 41
<212> DNA
<213> Artificial Sequence

<220>
<223> Description of Artificial Sequence:
      Primer

<400> 7
ggtgctgatt ggccccgcac gcctgacaat ctggtgtaac g 41

<210> 8
<211> 41
<212> DNA
<213> Artificial Sequence

<220>
<223> Description of Artificial Sequence:
      Primer

<400> 8
cgttacacca gattgtcagg cgtgcggggc caatcagcac c 41

<210> 9
<211> 52
<212> DNA
<213> Artificial Sequence

<220>
<223> Description of Artificial Sequence:
      Primer

<400> 9
gtagttaatt ttttaaaaag gtgcacgctt cctattggtc tgatttcaca gg 52

<210> 10
<211> 52
<212> DNA
<213> Artificial Sequence

<220>
<223> Description of Artificial Sequence:
      Primer

<400> 10
cctgtgaaat cagaccaata ggaagcgtgc acctttttaa aaaattaact ac          52


<210> 11
<211> 42
<212> DNA
<213> Artificial Sequence

<220>
<223> Description of Artificial Sequence:
      Primer

<400> 11
gcctctccct tctatgcacg tgcgaaagcc agcagcagcc cc                      42


<210> 12
<211> 42
<212> DNA
<213> Artificial Sequence

<220>
<223> Description of Artificial Sequence:
      Primer

<400> 12
ggggctgctg ctggctttcg cacgtgcata gaagggagag gc                      42

## Description

The present invention relates to nucleic acid molecules originally located within the IL-4 promoter which specifically interact with IRF-1 and/or IRF-2. The present invention also relates to pharmaceutical compositions containing said nucleic acid molecules.

Mature CD4⁺ T-lymphocytes can be divided into two distinct subpopulations named Th1 and Th2 based on their pattern of cytokines expression (Abbas et al., 1996; Mosmann et al., 1989). The Th1 subpopulation is characterized by the production of IFN-γ, IL-2, TNF and LT, cytokines involved in cell mediated immune responses such as delayed type hypersensitivity and macrophage activation. In contrast, the Th2 subpopulation produces IL-4, IL-5 and IL-10 and plays a major role in antibody-dependent immune responses such as allergy responses via activation of B-cells, mast cells and eosinophils (Mosmann et al., 1986; Paul et al., 1994). Th2 cytokines such as IL-4 are central to B-cells switching to IgE antibody production and IgE-dependent inflammatory reactions are generally characteristic of atopic disorders like asthma and rhinoconjunctivitis (Arthur et al., 1986).

Since the particular cytokines produced during an immune response determine recruitment and activation of other immune cells, the progression and outcome of autoimmune as well as infectious diseases is influenced by the cytokine milieu (Romagnani 1994; Lucey et al., 1996). The Th1 and Th2 subsets differentiate from native CD4⁺ Th precursor (Thp) cells. Significant progress has been made in understanding cytokines and transcription factors controlling differentiation of Th precursor cells into mature Th1 and Th2 cells. The critical Th2 inducing cytokine is IL-4 which mediates its effect on native Thp cells via the IL-4 receptor and the Stat6 signaling pathway (Kuhn et al., 1991; Kopf et al., 1993; Kaplan et al., 1996a; Shimoda et al., 1996; Takeda et al., 1996; O'Garra 1998). Transcription factors such as c-maf (Ho et al., 1996; 1998) and GATA3 (Zheng et al., 1997), along with NF-AT (Hodge et al., 1996a) and NIP45 (Hodge et al., 1996b) have been shown to play a major role in the transition towards Th2 cells. IL-12 exerting its effect via the IL-12 receptor and the Stat4 signaling pathway appear to be the major inducers of Th1 development (Kaplan et al., 1996b; Magram et al., 1996; Thierfelder et al., 1996). Furthermore, the transcription factor T-bet was found to be critically involved in murine Th1 development and in transactivation of the IFN-γ gene (Szabo et al., 2000).

The separation of mature CD4⁺ cells into Th1 and Th2 subsets is underscored by the fact that in many autoimmune and infectious diseases one T-helper cell phenotype is predominant (Romagnani, 1994). This predominance is attained by positive and negative feedback loops acting directly or indirectly between the two subsets. For example, IL-4 secreted by mature Th2 cells directly drives the polarization of native Thp towards Th2 cells (Paul et al., 1994), whereas IL-10, another Th2 cytokine, indirectly inhibits the polarization towards Th1 cells by repressing the secretion of IL-12 (the major Thl-inducing cytokine) by activated macrophages (Fiorentino et al., 1991). Moreover, IL-4 represses the expression of the IL-12 receptor on developing cells, screwing them towards Th2 cells (Szabo et al., 1997). Thus, Th2 cells reinforce their own phenotype while repressing the Th1 phenotype via IL-4 and IL-10. Recently, another feedback loop was added to the cytokine network by a study showing that in the murine system IL-4 promotes dendritic cells to produce bioactive IL-12, thereby causing differentiation of naive cells towards Th1 cells and providing a negative feedback on the Th2 phenotype (Holtschke et al., 1996). In contrast, Th1 cells promote their own subset via IFN-γ which induces IL-12 production from activated macrophages and the expression of the IL- 12 receptor on activated Thp cells (Szabo et al., 1997; Ma et al., 1996). However, so far feedback loops derived from Th1 cells that directly negatively regulate Th2 cells have not been described. To summarize, so far the regulation of the expression of IL-4 and factors involved are only poorly understood.

Thus, the technical problem underlying the present invention is to provide means for regulating the expression of IL-4 which might be useful as a medicament for treating disorders associated with an abnormal IL-4 expression.

The solution to said technical problem is achieved by providing the embodiments characterized in the claims.

In the experiments leading to the present invention it was investigated whether expression of the Th2 cytokine IL-4 is influenced by IFN-γ. It could be shown that IFN-γ directly down-regulates IL-4 expression in primary human T-lymphocytes. It was found that suppression of IL-4 expression by IFN-γ is mediated by IRF-1 and IRF-2. Previously, an IRF-2 binding site in the IL-4 promoter could be identified (Li-Weber et al., 1994). As shown in the Examples, below, two extra novel IRF binding sites could now be identified. One of these IRF-binding sites is located in the region which was previously shown to function as a T-cell specific negative regulatory element (Li-Weber et al., 1992). It could be shown here that IRF-2 and IRF-1 both function as repressors of the IL-4 promoter activity.

### BRIEF DESCRIPTION OF THE DRAWINGS

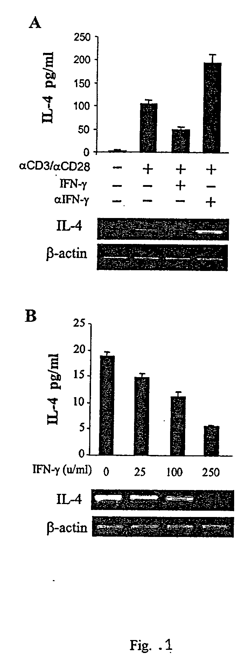

### Figure 1: Effect of IFN-γ on IL-4 secretion and IL-4 mRNA levels in activated primary human T-lymphocytes

(A) Primary human CD4⁺ T-lymphocytes were either untreated (-) or stimulated (+) with α-CD3 plus α-CD28 in the absence (-) or presence (+) of 100 U IFN-γ or 3 µg/ml IFN-γ neutralizing antibody as indicated. After 48 h culture, the supernatant was removed for measure IL-4 production by ELISA and the cells were collected for RNA isolation and subsequently for RT-PCR analysis of IL-4 mRNA levels. Comparable cell numbers were determined by Trypan blue exclusion.
(B) Primary human CD4-⁺T-lymphoeytes were treated as in (A) for 48 h in the presence of different concentrations of IFN-γ as indicated. The supernatant and the cells were analyzed for IL-4 production and IL-4 mRNA levels as in (A).

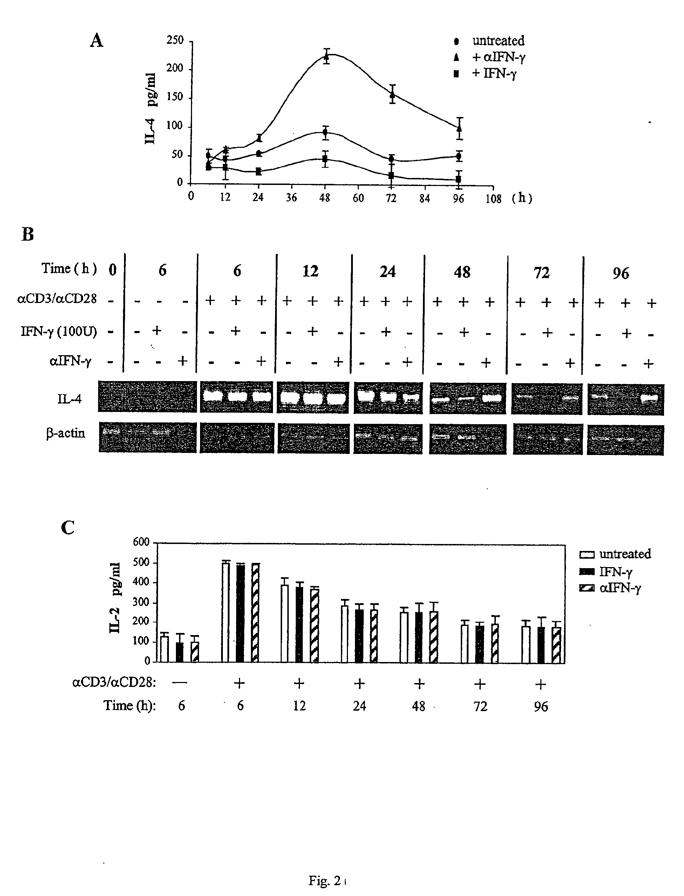

### Figure 2: Kinetic analysis of the influence of IFN-γ on IL-4 expression in primary human T-lymphocytes

(A) Primary human T-lymphocytes were stimulated with α-CD3 plus α-CD28 in the presence or absence of 100 U IFN-γ or 3 µg/ml IFN-γ neutralizing antibody for the indicated periods. The supernatant was removed for ELISA analysis of IL-4 protein. Results are representative of experiments conducted with cells from three donors. Mean and SD are shown.
(B) The cells treated in (A) were collected for RNA isolation and subsequently for RT-PCR analysis of IL-4 mRNA levels. - and + indicate without or with treatment as indicated.
(C) For control the specificity of the effect of IFN-γ on IL-4 production, the supernatant was also subjected for ELISA analysis of the IL-2 protein levels.

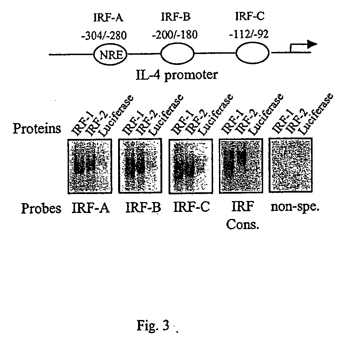

### Figure 3: Binding of in vitro translated IRF-1 and IRF-2 proteins to three distinct IL-4 promoter regulatory elements

IRF-1, IRF-2 and luciferase (as a control) proteins were prepared by in vitro transcription/translation and used in EMSA. The previously identified IL-4 IRF-2 binding site IRF-B and two novel potential IRF-binding sites, IRF-A [located in the previously identified negative-regulatory-element (NRE) region] and IRF-C, were used as probes. An oligonucleotide containing an IRF consensus (IRF Cons.) binding sequence and an unspecific oligonucleotide (non-spe.) were used as controls.

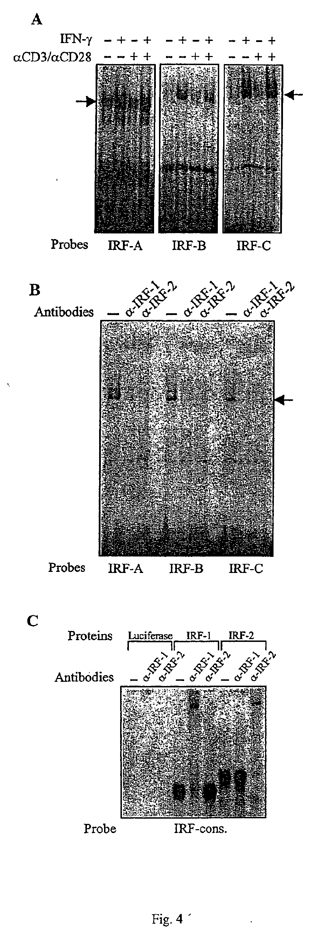

### Figure 4: Inducible binding of IRFs to the IL-4 IRF-binding sites upon IFN-γ stimulation

(A) EMSA was performed using nuclear extracts of primary human T-lymphocytes cultured for 24 h without (-) or with α-CD3 and α-CD28 antibodies in the absence or presence (+) of 100 U IFN-γ. The IL-4 IRF-A, IRF-B and IRF-C oligonucleotides were used as probes as indicated. The IFN-γ-inducible complexes are indicated by arrows.
(B) The IFN-γ-inducible complexes were analyzed by α-IRF-1 and α-IRF-2 specific antibodies. Nuclear extracts from primary human T-lymphocytes treated with IFN-γ for 24 h were used in EMSA. The specific IRF/DNA complexes and the supershifted bands are indicated by arrows.
(C) IFR-1 and IRF-2 proteins prepared from the in vitro transcription/translated system were used to control the specificity of the antibodies used. The IRF consensus sequence was used as the probe.

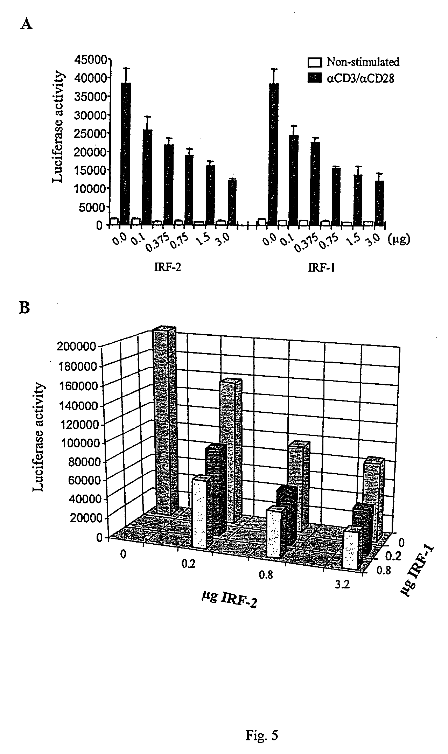

### Figure 5: Repression of IL-4 promoter activity by IRF-2 and IRF-1

Jurkat T cells were transiently transfected with an IL-4 promoter (-310/+11) luciferase construct together with the indicated amounts of either IRF-1 or IRF-2 expression vectors (A) or combinations of different amounts of IRF-1 and IRF-2 (B). The empty vector pCDNA3 was added to have equal amounts of DNA in the transfections. After overnight culture, the transfected cells were split and stimulated with α-CD3 and α-CD28 or left unstimulated. pRSV β-gal was used as an internal control for transfection efficiency. Luciferase and β-galactosidase activities were determined after 8 h of stimulation. The mean values and SD are representative for three independent experiments.

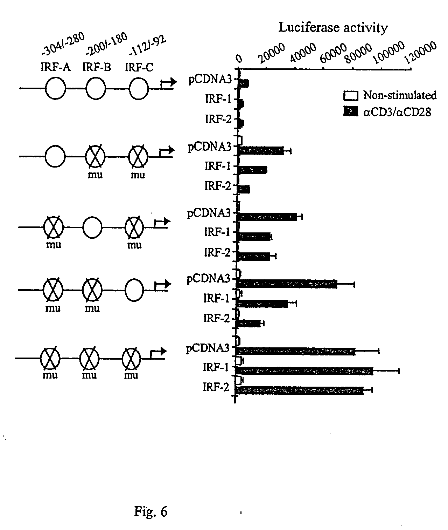

### Figure 6: Mutation analysis of the IL-4 promoter

The wild-type and the mutated IL-4 promoter constructs were cotransfected with 0.75 µg of either the IRF-1, the IRF-2 or the empty vector pCDNA3 into Jurkart T cells. The mutated sites are indicated in the left part of the figure. pRSV β-gal was included in all transfections to control transfection efficiency. After overnight culture, the transfected cells were split and stimulated with α-CD3 and α-CD28 or left unstimulated. Luciferase and β-galactosidase activities were determined after 8 h of stimulation. The mean values and SD are representative for three independent experiments.

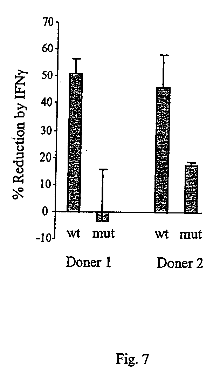

### Figure 7: Repression of IL-4 promoter by IFN-γ through the IRF sites

The wild-type (wt) and the IRF-mutated (mut) IL-4 promoter constructs were transfected into freshly isolated primary human T cells. After overnight culture, the transfected cells were split and stimulated with α-CD3 and α-CD28 in the absence or presence of 100 U IFN-y for further 8 h. Results from two different donors are presented. The men values and SD are representative for two independent transfections.

Accordingly, the present invention relates to a nucleic acid molecule capable of specifically interacting with IRF-1 and/or IRF-2, wherein said nucleic acid molecule comprises (a) the nucleic acid sequence 5'-TGG CCC CAA GTG ACT GAC AA-3' or 5'-CTA TGC AAA GCA AAA AGC CAG CAG CAG CCC CAA GCT-3', (b) a fragment of a nucleic acid sequence of (a) or (c) a nucleic acid sequence which hybridizes with a nucleic acid sequence of (a).

The nucleic acid molecules of the invention can be both DNA and RNA molecules. The nucleic acid molecules of the invention can be isolated from natural sources or can be synthesized according to known methods.

The present invention also provides nucleic acid molecules which hybridize to the above specific nucleic acid molecules. As used herein, the term "hybridize" has the meaning of hybridization under conventional hybridization conditions, preferably under stringent conditions as described, for example, in Sambrook et al., Molecular Cloning, A Laboratory Manual, 2^{nd} edition (1989) Cold Spring Harbor Laboratory Press, Cold Spring Harbor, NY.
Also contemplated are nucleic acid molecules that hybridize to the specific IRF-1/IRF-2 binding nucleic acid molecules at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO4; 0.02M EDTA, pH 7.4), 0.5% SDS, 30% formamide, 100 mg/ml salmon sperm blocking DNA, followed by washes at 50°C with 1 X SSPE, 0.1% SDS. In addition, to achieve even lower stringency, washes performed following stringent hybridization can be done at higher salt concentrations (e.g. 5X SSC). Variations in the above conditions may be accomplished through the inclusion and/or substitution of alternate blocking reagents used to suppress background in hybridization experiments. The inclusion of specific blocking reagents may require modification of the hybridization conditions described above, due to problems with compatibility.

In a further embodiment, the present invention provides nucleic acid molecules which comprise fragments of the specific IRF-1/IRF-2 binding nucleic acid molecules described above. "Fragments" are understood to be parts of the nucleic acid molecules that are long enough to still allow specific binding.

For the manipulation in prokaryotic cells by means of genetic engineering the nucleic acid molecules of the invention or fragments of these molecules can be introduced into plasmids allowing a mutagenesis or a modification of a sequence by recombination of DNA sequences. By means of conventional methods (cf. Sambrook et al., 1989, Molecular Cloning: A Laboratory Manual, 2^{nd} edition, Cold Spring Harbor Laboratory Press, NY, USA) bases can be exchanged and natural or synthetic sequences can be added. In order to link the DNA fragments with each other adapters or linkers can be added to the fragments. Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis method usually sequence analysis, restriction analysis and other biochemical or molecular biological methods are used.

The nucleic acid molecules of the present invention described above also comprise derivatives like PNAs. The nucleic acid molecules of the present invention (e.g., DNA, RNA or PNA) can bind to double-stranded or duplex IL-4 nucleic acids (e.g., in a region of the IL4-promoter region) forming a triple helix-containing, or "triplex" nucleic acid. Such triple helix formation might result in an increase of IL-4 expression.

Triplex oligo- and polynucleotides of the invention are constructed using the base-pairing rules of triple helix formation (see, e.g., Cheng et al., 1988, *J. Biol. Chem.* 263: 15110; Ferrin and Camerini-Otero, 1991, *Science* 354:1494; Ramdas et al., 1989, *J. Biol. Chem*. 264:17395; Strobel et al., 1991, *Science* 254:1639; and Rigas et al., 1986, *Proc. Natl. Acad. Sci. U.S.A*. 83: 9591) and the IL-4 promoter sequence. Typically, the triplex-forming oligonucleotides of the invention comprise a specific sequence of from about 10 to at least about 25 nucleotides or longer "complementary" to a specific sequence in the IL-4 promoter (i.e., large enough to form a stable triple helix, but small enough, depending on the mode of delivery, to administer *in vivo,* if desired). In this context, "complementary" means able to form a stable triple helix. For a review of recent therapeutic advances using triplex DNA, see Gee et al., in Huber and Carr, 1994, Molecular and Immunologic Approaches, Futura Publishing Co, Mt Kisco NY and Rininsland et al., 1997, *Proc. Natl. Acad. Sci. USA* 94:5854.

The invention furthermore relates to vectors containing the nucleic acid molecules of the invention. Preferably, they are plasmids, cosmids, viruses, bacteriophages and other vectors usually used in the field of genetic engineering. Vectors suitable for use in the present invention include, but are not limited to the T7-based expression vector for expression in bacteria, the pMSXND expression vector for expression in mammalian cells and baculovirus-derived vectors for expression in insect cells. Preferably, the nucleic acid molecule of the invention is operatively linked to the regulatory elements in the recombinant vector of the invention that guarantee the transcription and synthesis of an RNA in prokaryotic and/or eukaryotic cells that can be translated. The nucleotide sequence to be transcribed can be operably linked to a promoter like a T7, metallothionein I or polyhedrin promoter.

In a further embodiment, the present invention relates to recombinant host cells transiently or stably containing the nucleic acid molecules or vectors of the invention. A host cell is understood to be an organism that is capable to take up *in vitro* recombinant DNA and, if the case may be, to synthesize the RNAs encoded by the nucleic acid molecules of the invention. Preferably, these cells are prokaryotic or eukaryotic cells, for example mammalian cells, bacterial cells, insect cells or yeast cells. The host cells of the invention are preferably characterized by the fact that the introduced nucleic acid molecule of the invention either is heterologous with regard to the transformed cell, i.e. that it does not naturally occur in these cells, or is localized at a place in the genome different from that of the corresponding naturally occurring sequence.

The present invention also relates to a pharmaceutical composition comprising a nucleic acid molecule or vector of the present invention. For administration these compounds are preferably combined with suitable pharmaceutical carriers. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the disorder and the kind of compound contained in the pharmaceutical composition. The dosage regimen will be determined by the attending physian and other clinical factors. As is well known in the medical arts, dosages for any one patient depends on many factors, including the patient's size, body surface area, age, sex, the particular compound to be administered, time and route of administration, the kind of disorder, general health and other drugs being adminstered concurrently.

The above nucleic acids can be administered directly to the target site, e.g., by ballistic delivery, as a colloidal dispersion system or by catheter to a site in artery. The colloidal dispersion systems which can be used for delivery of the above nucleic acids include macromolecule complexes, nanocapsules, microspheres, beads and lipid-based systems including oil-in-water emulsions, (mixed) micelles, liposomes and lipoplexes. The preferred colloidal system is a liposome. The composition of the liposome is usually a combination of phospholipids and steroids, especially cholesterol. The skilled person is in a position to select such liposomes which are suitable for the delivery of the desired nucleic acid molecule. Organ-specific or cell-specific liposomes can be used in order to achieve delivery only to the target. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific targets via specific cell-surface ligands. By delivering these nucleic acids to the desired target, IRF-1 and/or IRF-2 molecules can be trapped. Accordingly, the intracellular expression of IL-4 and, thus, the level of IL-4 can be increased resulting in the enhancement of the positive effects of IL-4.

The delivery of the nucleic acid molecules of the invention can also be achieved by using a recombinant expression vector such as a chimeric virus. Preferred recombinant vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotid encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957.

In order to achieve expression only in the target organ, the nucleic acids of the invention can also be operably linked to a tissue specific promoter and used for gene therapy. Suitable tissue specific promoters are well known to those skilled in the art.

In a further embodiment, the present invention relates to the use of the nucleic acid molecules and vectors, respectively, of the present invention for the preparation of a medicament for the treatment of a disease associated with a decreased IL-4 expression.

Finally, the present invention also relates to the use of IRF-1, IRF-2 and/or IFN-γ for the preparation of a medicament for the treatment of a disease in which inhibition of IL-4 expression has a beneficial effect, e.g. an atopic disorder like asthma and rhinoconjuctivitis or an autoimmune disease.

The following examples illustrate the invention.

### Example 1

### General methods

### (A) Purification of primary human T-lymphocytes and CD4⁺ T-lymphocytes

Human peripheral T cells were prepared and activated as described previously (Klas et al., 1993). Purification of CD4⁺ T-lymphocytes was performed with the MACS CD4 Multisort Kit (Miltenyi Biotec, Bergisch Gladbach, Germany) according to the manufacturers instructions. Purity (>95%) was confirmed by FACS staining with a-CD4 antibodies (BD-Pharmingen, Heidelberg, Germany).

### (B) Culture and stimulation of primary human T-lymphocytes and cell lines

Primary human T-lymphocytes and Jurkat T cells were cultured in RPMI supplemeented with 10% heat-inactivated fetal calf serum (Gibco BRL, Eggenstein, Germany), 10mM HEPES (Gibco BRL, Eggenstein, Germany) and 100µg gentamycin/ml. Stimulation was performed by 3 µg/ml a-CD3 (OKT3) and 1,5 µg/ml a-CD28 crosslinked by 1µg/ml Protein A ( Sigma-Aldrich, Taufkirchen, Germany). Human IFN-γ and monoclonal a-human IFN-γ antibody were purchased from Roche Diagnostics GmbH (Mannheim, Germany) and R&D Systems GmbH (Wiesbaden, Germany ), respectively.

### (C) ELISA

Supernatants from cultures treated as described in the text were measured for cytokine levels by ELISA using ELISA kits for human IL-4 (BD-Pharmingen, Heidelberg, Germany) according to the manufacturers instructions.

### (D) RT-PCR

RNA was isolated using the RNEasy kit (Qiagen GmbH, Hilden, Germany) according to the manufactures instructions. 1 µg of total RNA was reverse transcribed using the Perkin Elmer GeneAmp RNA PCR kit (Foster City, California). Aliquots were amplified in a DNA thermocycler (Stratagene, Heidelberg, Germany) with 2.5 U recombinant Taq polymerase (Sigma, St.Louis, Missouri) in a 50 µl reaction. Thirty cycles were performed each consisting of the following conditions: 94 °C 1min 56 °C 1 min and 72 °C 1 min. Primers for human IL-4 and b-actin have been described previously (Li-Weber et al 1998). Amplification products were separated by electrophoresis on 1.2% agarose gels.

### (E) Preparation of IRF-1 and IRF-2 protein

TNT Quick Coupled Transcription/Translation systems (Promega Corporation, Madison, Wisconsin) was used to obtain *in vitro* translated IRF-1 and IRF-2 protein according to manufactures instructions. IRF-1 and IRF-2 plasmids used for the *in vitro* transcription/translation were published previously (Harada et al., 1994; Kiechhoff et al., 1993).

### (F) EMSA

Nuclear extracts were prepared as described previously (Li-Weber et al., 1994). The following oligonucleotides were used as probes in EMSA:

-92/-112 TGG CCC CAA GTG ACT GAC AA,

-180/-200 AAA GGT TTC ATT TTC CTA TT,

-280/-304 CTA TGC AAA GCA AAA AGC CAG CAG CAG CCC CAA GCT,
IRF consensus TCACTTTCACTTTCACTT.

For binding reactions 0.5 µg of nuclear extract was incubated with reaction buffer ((Li-Weber et al., 1994)) in the presence or absence of antibody for 10 min at room temperature. Then the radiolabeled probe (≥ 20000 cpm) was added and incubated for an additional 20 min. The binding products were analyzed by electrophoresis on 5 % polyacrylamide gel in 0.5xTBE buffer. Gels were dried and analyzed by autoradiography.

### (G) Plasmid construction

PLuc-IL4 -310/+11 was constructed by cutting the -310/+11 IL-4 fragments from the CAT-IL4 -310/+11 construct (28) and recloning into the multiple-cloning site of the pLuc plasmid (Kindly supplied by Dr. T. Wirth, Institute of Medicine and Cell research, Würzburg, Germany). Mutations were performed using the QuikChange mutagenesis kit (Stratagene, La Jolla, California) according to the manufactures instructions. The primers used for mutations were:

IRF-A mu-s 5`-GGTGCTGATTGGCCCCGCACGCCTGACAATCTGGTGTAACG-3`,

IRF-A mu-a  5`-CGTTACACCAGATTGTCAGGCGTGCGGGGCCAATCAGCACC-3`,

IRF-B mu-s 5`-GTAGTTAATTTTTTAAAAAGGTGCACGCTTCCTATTGGTCTGATT
TCACAGG-3`,

IRF-B mu-a 5`-CCTGTGAAATCAGACCAATAGGAAGCGTGCACCTTTTTAAAAAAT
TAACTAC-3`,

IRF-C mu-s 5`-GCCTCTCCCTTCTATGCACGT GCGAAAGCCAGCAGCAGCCCC-3`,

IRF-C mu-a 5`-GGGGCTGCTGCTGGCTTTCGCACGTGCATAGAAGGGAGAGGC-3`.

Underlined nucleotides represent mutated sites. All mutations were confirmed by sequence analysis. The IRF-1 (Miyamoto et al., 1988) and IRF-2 (Harada et al., 1994) expression vectors were kindly supplied by Dr. T. Tanigushi (Osaka university, Japan).

### (H) Transient transfections

Jurkat T cells were transiently transfected by electroporation. In all transfection experiments 10µg of reporter construct were transfected using a Bio-Rad Gene Pulser (Bio-Rad GmbH, München, Germany) set at 960 microfarads, 240 V. To transfect primary human T-lymphocytes DOTAP Liposomal Transfection Reagent (Roche Diagnostics GmbH, Mannheim, Germany) was used according to the manfactures instructions. To control for transfection efficiency, cells were cotransfected with 1µg of pRSV b-gal (a kind gift from Dr. L. Schmitz, German Cancer Research Center, Germany). Luciferase activity was determined in 10 ml of cell extract using the luciferase assay substrate (Promega, Mannheim, Germany) with an Duolumat LB9507 luminometer (Berthold, Freiburg, Germany). b-galactosidase activity was determined by incubation of 25 ml of cell extracts in buffer (final volume 300 ml) containing 100 mM Na₂HPO₄, 100 mM NaH₂PO₄, PH 7.5, 1 mM MgCl₂, 45 mM b-Mecaptoethanol, 1 mg/ml o-nitrophenyl-b-D-galactopyranoside and 1 ml b-galactosidase) at 37 °C for 30 min. The reaction was stopped by adding 500 ml 1 M Na₂CO₃ and measured by photometer at OD600.

### Example 2

### IFN-γ downregulates IL-4 protein and mRNA levels

IFN-γ is the signature cytokine of Th1 cells and promotes the polarization of naive Th cells towards Th1 cells. Therefore, we wondered whether IFN-γ influenced the level of IL-4 expression. To clarify this question, we isolated primary human peripheral blood T-lymphocytes of healthy donors and activated the cells via TCR/CD3 and CD28 in the absence or presence of IFN-g. The supernatants were analyzed for IL-4 production by ELISA and the cells were collected for determination of IL-4 mRNA expression levels. Since primary human T-lymphocytes produce large amounts of IFN-γ upon activation, we also added IFN-γ neutralizing antibodies into the medium to investigate the effect of IFN-γ on IL-4 production. As shown in Fig. 1, IL-4 protein secretion was induced upon activation of the primary T cells by a-CD3 and a-CD28. In the presence of IFN-γ, IL-4 secretion was significantly reduced. In contrast, in the presence of the IFN-g neutralizing antibodies IL-4 production was increased (Fig. 1A). To test whether the reduced IL-4 protein levels in the supernatants were due to reduction of IL-4 transcripts we measured IL-4 mRNA levels. As shown in Fig. 1A, IL-4 mRNA levels were reduced by addition of INF-γ but increased in the presence of IFN-γ neutralizing antibodies. Secretion of the IL-4 protein and expression of the IL-4 mRNA were repressed by IFN-γ in a dose-dependent manner (Fig. 1B). The decreased IL-4 levels were not due to a decrease in cell numbers. Trypan Blue exclusion showed comparable numbers of survival cells in all groups. The IFN-γ induced repression of IL-4 mRNA and IL-4 protein production also did not depend on CD28 co-stimulation. Stimulation of primary T-lymphocytes with α-CD3 antibody alone showed a similar reduction of IL-4 by IFN-γ (data not shown).

To further investigate the effect of INF-γ on IL-4 expression, we performed kinetics. At the initial phase of T cell activation, no influence of IFN-γ on IL-4 protein expression levels was seen. However, reduction of IL-4 became pronounced after 24 h activation of the T-lymphocytes in the presence of IFN-g (Fig. 2A). Correspondingly, IL-4 mRNA levels showed similar kinetics as those observed on the IL-4 protein expression level (Fig. 2B). IFN-γ induced repression of IL-4 mRNA expression lasted at least up to 96 h post stimulation. The IFN-γ induced repression was specific for IL-4 since e. g. IL-2 levels were not affected (Fig. 2C). Taken together, these data demonstrate that IFN-γ can repress IL-4 production in peripheral T cells.

### Example 3

### IRF-1 and IRF-2 bind to three sites of the IL-4 promoter

Since IL-4 mRNA levels were reduced in the presence of IFN-γ, we further investigated the molecular mechanism of IFN-γ-mediated suppression. Previously, we have identified a binding site for the constitutive factor IRF-2 at the -200/-180 region (referred to hereafter as IRF-B site) in the IL-4 promoter. Mutation at this site resulted in a two-fold increase in promoter activity upon PMA/ionomycin or PMA/PHA stimulation of Jurkat T cells (Li-Weber et al., 1994). Therefore, we reasoned that binding of IRFs to the IL-4 promoter may be a possible mechanism for IFN-γ-mediated IL-4 repression. Besides the IRF-B site, we further identified two additional potential IRF binding sites located in the -304/-280 region (referred as IRF-A site) and the -112/-92 (referred as IRF-C site) of the IL-4 promoter, respectively. Interestingly, IRF-A is located within the region which was previously shown to exert a T cell-specific negative effect on IL-4 promoter activity (Li-Weber et al., 1992). To investigate whether these potential IRF sites are recognized by IRF proteins, we used *in vitro* translated IRF-1 and IRF-2 proteins in EMSA. The specificity of the IRF-1 and IRF-2 proteins was controlled by using a DNA probe containing the consensus IRF site and an unrelated DNA fragment as a negative control. As shown in Fig. 3, IRF-1 and IRF-2 proteins bind to all three promoter sites indicating that all three sites interact with IRF proteins.

We next investigated whether the IL-4 IRF sites interact with IRFs induced by IFN-γ in activated T cells. Nuclear proteins isolated from freshly isolated human blood T-lymphocytes untreated or stimulated by α-CD3 and α-CD28 in the presence or absence of IFN-γ were used in EMSA. IFN-γ-inducible binding activities were found on all three DNA probes (Fig. 4A). In contrast, activation of T lymphocytes with α-CD3 and α-CD28 antibodies alone did not influence the binding activity. INF-γ induced complexes were supershifted by α-IRF-1 and α-IRF-2 antibodies demonstrating that IRF-1 and IRF-2 bind to these sites (Fig. 4B). The specificities of the α-IRF-1 and α-IRF-2 antibodies were controlled by the *in vitro* translated IRF proteins in EMSA. As shown in Fig. 4C, the IRF-1 and IRF-2 antibodies specifically supershifted IRF-1 and IRF-2, respectively. These experiments demonstrate that the IL-4 promoter contains at least three IRF binding sites.

### Example 4

### IRF-1 and IRF-2 act as repressors of the IL-4 promoter

The finding of three IRF binding sites prompted us to further investigate the functional role of IRF-1 and IRF-2 in the context of IL-4 promoter activity. A luciferase reporter construct containing up to -310 of the human IL-4 promoter was used for transient transfection studies. Cotransfection into Jurkat T cells of the IL-4 reporter construct together with expression vectors containing either the IRF-1 or the IRF-2 cDNA or the vector alone showed a dose dependent repression of the IL-4 promoter (Fig. 5A). Cross-titrations of IRF-1 and IRF-2 also showed a persistent repression for all combinations of IRF-1 and IRF-2 (Fig. 5B). The repression was dependent on the absolute amount of IRFs, but not on the ratio of IRF-1 and IRF-2. The experiments demonstrate that both IRF-2 and IRF-1 can act as repressors in the context of the IL-4 promoter.

We further investigated the functional importance of the IRF sites on the IL-4 promoter and introduced different combinations of mutations into these sites. IRF-2 is constitutively present in Jurkat T cells (Li-Weber et al., 1994). Transfection of the wild-type and the mutated IL-4 promoter constructs into Jurkat T cells showed that mutation of the IRF sites greatly enhanced the activity of the IL-4 promoter in response to T cell activation (Fig. 6). Cotransfection of the promoter constructs with IRF expression vectors showed that each IRF site responded to IRF-1- and IRF-2-mediated repression. When all three IRF sites were mutated, the promoter activity was no longer downregulated by IRFs (Fig. 6). These data demonstrate that all three sites are functional IRF sites and IRFs repress IL-4 promoter activity through these three sites.

To further investigate whether the IRF sites are required for INF-γ-mediated suppression of IL-4 transcription the wild-type and the three IRF-mutated IL-4 promoter constructs, respectively, were transfected into freshly isolated primary human T-lymphocytes using a liposome based transfection system. Fig. 7 shows the results from two different T cell donors. In the presence of IFN-g, the activity of the wild-type IL-4 promoter reporter constructs was reduced by approximately 45-50%. In contrast, the activity of the IL-4 promoter containing mutations of all three IRF sites was substantially less affected by IFN-γ. The experiments demonstrate that all three IRF binding sites are involved in the IFN-γ-mediated suppression of IL-4 expression in normal T cells.

### REFERENCES

Abbas, A.K., Murphy, K.M., and Sher, A. (1996). Functional diversity of helper T lymphocytes. Nature *383,* 787-793.
Arthur, R.P. and Mason, D. (1986). T cells that help B cell responses to soluble antigen are distinguishable from those producing interleukin 2 on mitogenic or allogeneic stimulation. J. Exp. Med. *163,* 774-786.
Bluyssen, H.A.R., Durbin, J.E. and Levy, D.E. (1996). ISGF3g p48, a specificity switch for interferon activated transcription factors. Cyt. Growth Fact. Rev. 7, 11-17.
Driggers, P.H., Ennist, D.L., Gleason, S.L., Mak, W-H., Marks, M.S., Levi, B-Z., Flanagan, J.R., Appella, E., Ouato, K. (1990). An interferon g-regulated protein that binds the interferon-inducible enhancer element of major histocompatibility complex class I genes. Proc. Natl. Acad. Sci. USA *87*, 3743-3747.
Fiorentino, D.F., Zlotnik, A., Vieira, P., Mosmann, T., Howard, M., Moore, D.W., and OGarra, A. (1991). IL-10 acts on the antigen-presenting cell to inhibit cytokine production by Th1 cells. J. Immunol. *146,* 3444-3451.
Harada, H., Willison, K., Sakakibara, J., Miyamoto, M., Fujita, T. and Taniguchi, T. (1990). Absence of type I IFN system in EC cells: transcriptional activator (IRF-1) and repressor (IRF-2) genes are developmentally regulated. Cell *63,* 903-913.
Harada, H., Takahashi, E., Itoh, S., Harada, K., Hori, T.A. and Taniguchi, T. (1994). Structure and regulation of the human interferon regulatory 1 (IRF-1) and IRF-2 genes: implications for a gene network in the interferon system. Mol. Cell. Biol. *14*:1500-1509.
Ho, I.-C., Hodge, M.R., Rooney, J.W., and Glimcher, L.H. (1996). The proto-oncogene c-maf is responsible for tissue-specific expression of interleukin-4. Cell *85*, 973-983.
Ho, I.-C., Lo, D., and Glimcher, L.H. (1998). C-maf promotes Th2 and attenuates Th1 differentiation by both IL-4 dependent and independent mechanisms. J. Exp. Med. *188,* 1859-1866.
Hochrein, H., O'Keeffe, M., Luft, T., Vandenabeele, S., Grumont, R.J., Maraskovsky, E., and Shortman, K. (2000), Interleukin (IL)-4 is a major regulatory cytokine governing bioactive IL-12 production by mouse and human dendritic cells, J. Exp. Med. *192*, 823-834.
Hodge, M.R., Ranger, A.M., Charles de la Brousse, F., Hoey, T., Grusby, M.J., and Glimcher, L.H. (1996a). Hyperproliferation and dysregulation of IL-4 expression in NF-ATp-deficient mice. Immunity *4*, 1-20.
Hodge, M.R., Chun, H.J., Rengarajan, J., Alt, A., Lieberson, R., and Glimcher, L.H. (1996b). NFAT-driven interleukin-4 transcription potentiated by NIP45. Science *274*, 1903-1905.
Holtschke, T., Löhler, J., Kanno, Y., Fehr, T., Giese, N., Rosenbauer, F., Lou, J., Knobeloch, K.P., Gabriele, L., Waring, J.F., Bachmann, M.F. and Zingernagel, R.M. (1996). Immunodeficiency and chronic myelogenous leukemia-like syndrome in mice with a targeted mutation of the ICSBP gene. Cell *87*, 307-317.
Kaplan, M.H., Schindler, U., Smiley, S.T., and Grusby, M.J. (1996a). Stat6 is required for mediating responses to IL-4 and for the development of Th2 cells. Immunity 4, 313-319.
Kaplan, M.H., Sun, Y.L., Hoey, T., and Grusby, M.J. (1996b). Impaired IL-12 responses and enhanced development of Th2 cells in Stat4-deficient mice. Nature *382,* 174-177.
Kimura, T., Kadokawa, Y., Harada, H., Matsumoto, M., Sato, M., Kashiwazaki, Y., Tarutani, M., Tan, R.S.P., Takasugi, T., Matsuyama, T., Mak, T.M., Noguch, S. and Taniguchi, T. (1996). Essential and non-redundant roles of p48 (ISGF3g) and IRF-1 in both type I and type II interferon responses, as revealed by gene targeting studies. Genes Cells *1*, 115-124.
Kirchhoff, S., Schaper, F. and Hauser, H. (1993). Interferon regulatory factor 1 (IRF-1) mediates cell growth inhibition by transactivation of downstream target genes. Nucl. Acid. Res. *21*, 2881-2889.
Kirchhoff, S., Oumard, A., Nourbakhsh, M., Levi, B. Z. and Hauser, H. (2000). Interplay between repressing and activating domains defines the transcriptional activity of IRF-1. Eur. J. Biochem. *267*, 6753-6761.
Klas C, Debatin KM, Jonker RR, Krammer PH. (1993). Activation interferes with the APO-1 pathway in mature human T cells. Int Immunol. *5*, 625-630.
Kopf, M., Le Gros, G., Bachmann, M., Lamers, M.C., Bluethmann, H., and Kohler, G. (1993). Disruption of the murine IL-4 gene blocks Th2 cytokine responses. Nature *362,* 245-248.
Kuhn, R., Rajewsky, K., and Muller, W. (1991). Generation and analysis of interleukin-4 deficient mice. Science *254,* 707-710.
Li-Weber, M., Eder, A., Krafft-Czpa, H. and Krammer, P. H. (1992). T cell-specific negative regulation of transcription of the human cytokine IL-4. J. Immunol. *148,* 1913-1918.
Li-Weber, M., Davydov, I.V., Krafft, H. and Krammer, P.H. (1994). The role of NF-Y and IRF-2 in the regulation of human IL-4 gene expression. J. Immunol. *153,* 4122-4133.
Li-Weber, M., Giasi, M., and Krammer, P.H. (1998), Involvement of Jun and Rel proteins in up-regulation of interleukin-4 gene activity by the T -cell accessory molecule CD28, J. Biol. Chem. *273*, 32460-32466.
Lohoff, M., Ferrick, D., Mittrucker, H.W., Duncan, G.S., Bischof, S., Rollinghoff, M., and Mak, T.W. (1997). Interferon regulatory factor-1 is required for a T helper 1 immune response in vivo. Immunity **6**, 681-689.
Lohoff, M., Duncan, G.S., Ferrick, D., Mittrucker, H.W., Bischof, S.,Prechtl, S., Rollinghoff, M., Schmitt, E., Pahl, A. and Mak, T.W. (2000). Deficiency in the transcription factor interferon regulatory factor-2 ( IRF-2 ) leads to severly compromised development of natural killer and T-helper cells. J. Exp. Med. *192*, 325-335.
Lucey, D.R., Clerici, M. and Shearer, G.M. (1996). Type 1 and type 2 cytokine dysregulation in human infectious, neoplastic, and inflammatory diseases. Clin. Microbiol. Rev. *9*, 532-562.
Ma, X., Chow, J.M., Gri, G., Carra, G., Gerosa, F., Wolf, S.F., Dzialo, R., Trinchieri, G. (1996). The interleukin 12 p40 gene promoter is primed by interferon gamma in monocytic cells. J. Exp. Med *183,* 147-157.
Magram, J., Connaughton, S.E., Warrier, R.R., Carvajal, D.M., Wu, C., Ferrante, J., Stewart, C., Sarmiento, U., Faherty, D.A., and Gately, M.K. (1996). IL-12-deficient mice are defective in IFN production and type 1 cytokine responses. Immunity *4*, 471-481.
Mamane, Y., Heylbroeck, C., Cenin, P., Algarte, M., Servant, M. J., LePage, C., DeLuca, C., Kwon, H., Lin, R. and Hiscott, J. (1999). Interferon regulatory factors: the next generation. Gene 237, 1-14.
Matsuyama, T., Kimura, T., Kitagawa, M., Pfeffer, K., Kawakami, T., Watanabe, N., Kundig, T.M., Amakawa, R., Kishihara, K., and Wakeham, A. (1993). Targeted disruption of IRF-1 or IRF-2 results in abnormal type I IFN gene induction and aberrant lymphocyte development. Cell 75, 83-97.
McElliot, D.L., Phillips, J.A., Stillman, C.A., Koch, R.J., Moiser, D.E. and Hobbs, M.V. (1997). CD4+ T cells from IRF-1-deficient mice exhibit altered patterns of cytokine expression and cell subset homeostasis. J. Immunol. *159,* 4180-4186.
Miyamoto, M., Fujita, T., Kimura, Y., Maruyama, M, Harada, H., Sudo, Y., Miyata, T. and Taniguchi, T. (1988). Regulated expression of a gene encoding a nuclear factor, IRF-1, that specifically binds to IFN-b gene regulatory elements. Cell *54*, 903-913.
Mosmann, T.R., Cherwinski, H., Bond, M.W., Giedlin, M.A., and Coffman, R.L. (1986). Two types of murine helper T cell clone. I. Definition according to profiles of lymphokine activities and secreted proteins. J. Immunol. *136,* 2348-2357.
Mosmann, T.R. and Coffman, R.L. (1989). TH1 and TH2 cells: Different patterns of lymphokine secretion lead to different functional properties. Annu. Rev. Immunol. *7*, 145-173.
Nelson, N., Kanno, Y., Hong, C., Contursi, C., Fujita, T., Fowlkes, B.J., O'Connell, E., Hu-Li, J., Paul, W.E., Jankovic, D., Sher, A.F., Coligan, J.E., Thornton, A., Appella, E., Yang, Y. and Ozato, K. (1996). Expression of IFN regulatory factor family proteins in lymphocytes. Induction of Stat-1 and IFN consensus sequence binding protein expression by T cell activation. J. Immunol. *156,* 3711-3720.
O'Garra, A. (1998). Cytokines induce the development of functionally heterogeneous T helper cell subsets. Immunity *8*, 275-283.
Paul, W.E. and Seder, R.A. (1994). Lymphocyte responses and cytokines. Cell *76*, 241-251
Romagnani, S. (1994). Lymphokine production by human T cells in disease states. Annu Rev Immunol. *12,* 227-57.
Sharf, R., Azriel, A., Lejbkowicz, F., Winograd, S.S., Ehrlich, R. and Levi, B.Z. (1995). Functional domain analysis of interferon consensus sequence binding protein (ICSBP) and its association with interferon regulatory factors. J. Biol. Chem. *270*, 13063-13069.
Shimoda, K., van Deursen, J., Sangster, M.Y., Sarawar, S.R., Carson, R.T., Tripp, R.A., Chu, C., Quelle, F.W., Nosaka, T., Vignali, D.A. (1996). Lack of IL-4-induced Th2 response and IgE class switching in mice with disrupted Stat6 gene. Nature *380,* 630-633.
Stark, G.R., Kerr, I.M., Williams, B.R.G., Silverman, R.H. and Schreiber, R.D. (1998). How cells respond to interferons. Annu. Rev. Biocem. *67*, 227-264.
Szabo, S.J., Kim, S.T., Costa, G.L., Zhang, X., Fathman, G.C., and Glimcher, L.H. (2000). A novel transcription factor, T-bet, directs Th1 lineage commitment. Cell *100,* 655-669.
Szabo, S.J., Dighe, A.S., Gubler, U., and Murphy, K. (1997). Regulation of the interleukin (IL)-12R 2 subunit expression in developing T helper 1 (Th1) and Th2 cells. J. Exp. Med. *185*, 817-824.
Takeda, K., Tanaka, T., Shi, W., Matsumoto, M., Minami, M., Kashiwamura, S., Nakanishi, K., Yoshida, N., Kishimoto, T., and Akira, S. (1996). Essential role of Stat6 in IL-4 signalling. Nature *380,* 627-630.
Taki, S., Sato, T., Ogasawara, K., Fukuda, T., Sato, M., Hida, S., Suzuki, G., Mitsuyama, M., Shin, E.H., Kojima, S., Taniguchi, T., and Asano, Y. (1997). Multistage regulation of Th1-type immune responses by the transcription factor IRF-1. Immunity 6, 673-679.
Tanaka, N. and Taniguchi, T. (2000). The interferon regulatory factors and oncogenesis. Semi. Cancer Biol. *10*, 73-81.
Thierfelder, W.E., van Deursen, J.M., Yamamoto, K., Tripp, R.A., Sarawar, S.R., Carson, R.T., Sangster, M.Y., Vignali, D.A., Donherty, P.C., Grosveld,G.C., and Ihle, J.N. (1996). Requirement for Stat4 in interleukin-12-mediated responses of natural killer and T cells. Nature *382,* 171-174.
Vaughan, P.S., Aziz, F., van Wijnen, A.J., Wu, S., Harada, H., Taniguchi, T., Soprano, K J., Stein, J.L. and Stein, G.S. (1995). Activation of a cell-cycle-regulated histone gene by the oncogenic transcription factor IRF-2. Nature 377, 362-365.
Vaughan, P.S., Van Der Meijden, C., Aziz, F., Jarada, H., Taniguchi, T., Van Wijnen, A., Stein, J.L. and Stein, G.S. (1998). Cell cycle regulation of histone H4 gene transcription requires the oncogenic factor IRF-2. J. Biol. Chem. *273*, 194-199.
Zheng, W.-P. and Flavell, R.A. (1997). The transcription factor GATA-3 is necessary and sufficient for Th2 cytokine gene expression in CD4 T cells. Cell *89*, 587-596.

## Claims

1. A nucleic acid molecule capable of specifically interacting with IRF-1 and/or IRF-2, wherein said nucleic acid molecule comprises (a) the nucleic acid sequence 5'-TGG CCC CAA GTG ACT GAC AA-3' or 5'-CTA TGC AAA GCA AAA AGC CAG CAG CAG CCC CAA GCT-3', (b) a fragment of a nucleic acid sequence of (a) or (c) a nucleic acid sequence which hybridizes with a nucleic acid sequence of (a).

2. A vector containing a nucleic acid molecule of claim 1.

3. A pharmaceutical composition containing a nucleic acid molecule of claim 1 or a vector of claim 2.

4. Use of a nucleic acid molecule of claim 1 or a vector of claim 2 for the preparation of a medicament for the treatment of a disease associated with a decreased IL-4 expression.
